(19) Europäisches Patentamt
European Patent Office
Office européen des brevets

(11) EP 2 008 582 A1

(12) **EUROPEAN PATENT APPLICATION**
published in accordance with Art. 153(4) EPC

(43) Date of publication:
**31.12.2008 Bulletin 2009/01**

(21) Application number: **07739315.5**

(22) Date of filing: **22.03.2007**

(51) Int Cl.:
***A61B 5/022*** *(2006.01)*

(86) International application number:
**PCT/JP2007/055872**

(87) International publication number:
**WO 2007/116635 (18.10.2007 Gazette 2007/42)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **12.04.2006 JP 2006110093**

(71) Applicant: **Omron Healthcare Co., Ltd.**
**Kyoto 615-0084 (JP)**

(72) Inventor: **SHIRASAKI, Osamu**
**Kyoto-shi, Kyoto 615-0084 (JP)**

(74) Representative: **Wilhelms · Kilian & Partner**
**Patentanwälte**
**Eduard-Schmid-Strasse 2**
**81541 München (DE)**

# (54) BLOOD PRESSURE METER WHERE VARIATION IN BLOOD PRESSURE VALUE CAN BE KNOWN

(57) In a sphygmomanometer (1), a measurement value in a blood pressure measurement unit (16) and a measurement time are corresponded and stored in a blood pressure storage unit (17). When a measurement data process program is executed in CPU (11), the measurement value is extracted from the blood pressure storage unit based on a time the program is executed. The extracted measurement values in a plurality of measurement dates are integrated, and are used for a process of calculating a blood pressure average value for every period of time, and a process for obtaining a blood pressure variation curve.

Fig. 1

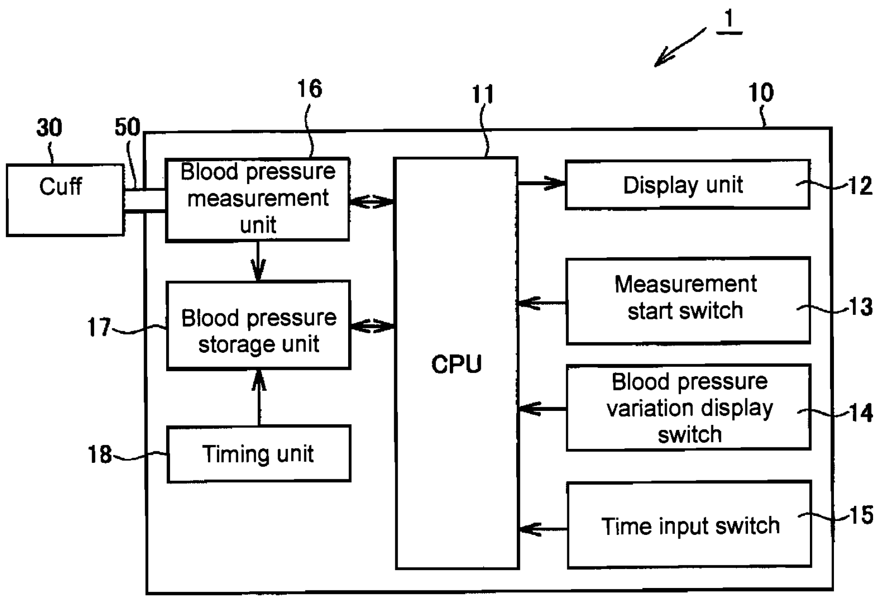


EP 2 008 582 A1

## Description

TECHNICAL FIELD

The present invention relates to sphygmomanometers, blood pressure measurement systems, and measurement data processing program products, in particular, to a sphygmomanometer capable of knowing variation in blood pressure values, a blood pressure measurement system, and a measurement data processing program product.

BACKGROUND ART

The blood pressure is well known to vary greatly during the day. The variation in blood pressure during the day is referred to as the circadian variation in the blood pressure. The pattern of the circadian variation greatly varies between individuals. It is recently known that the variation pattern specific to the individual exists during one week. It has become apparent by many researches that the rate of crisis of high-blood pressure disease becomes high depending on the variation pattern.

For instance, early morning hypertension is a clinical condition showing a circadian variation pattern in which the blood pressure is usually normal, but specifically high one to two hours before and after wakeup. The nocturnal hypertension is a clinical condition in which the blood pressure during sleep is high. Both of which are considered risk factors of brain or heart disease, and are considered to be strongly associated with sudden death. The office hypertension, which is recently given attention, refers to a state in which the blood pressure is specifically high only during the day of the working days, and take a normal value in non-working days such as weekends or at night. Such blood pressure variation pattern also changes by days, which is referred to as daily variation. For instance, Monday surge refers to a state in which the blood pressure is high particularly on the first day of work after vacation.

Thus, there are various blood pressure variation patterns, and consideration is starting to be made that measuring the blood pressure and understanding the variation pattern thereof are useful in diagnosis/treatment of high blood pressure. In other words, the true clinical condition of the patient cannot be understood by simply measuring the blood pressure at one point in the outpatient service as in the prior art. If the variation pattern of the blood pressure can be acquired, higher quality hypertension treatment can be expected by adjusting the prescription of hypotensive drug, specifically, amount of medicine, time to take medicine, and the like.

Generally, an ambulatory blood pressure monitor (hereinafter referred to as ABPM) is used to acquire the variation pattern of the blood pressure. The ABPM is a monitor having a configuration of attaching a cuff on the upper arm, and periodically measuring /recording blood pressure with a compact automatic sphygmomanometer which is constantly carried around.

[Patent document 1] Japanese Laid-Open Patent Publication No. 2004-261452

[Non-patent document 1] Machiko Take et al. "Reliability of portable blood pressure continuous measurement device ABPM-630 and reproducibility of blood pressure circadian variation", Journal of Tokyo Women's Medical University, May, 1990, No. 60(5) pp. 430 to 437.

DISCLOSURE OF THE INVENTION

PROBLEMS TO BE SOLVED BY THE INVENTION

However, this method has many problems described below.

First, the ABPM is expensive, and cannot be purchased by an individual for daily use. Thus, normally, it is purchased by a medical facility and lent to a patient for use. However, as it needs to be lent to many patients, the lending period to one patient is limited. Thus, the measurement is normally limited to one day or two days at the most. Since the variation pattern of the blood pressure has daily variation, as described above, the blood pressure data of the day the measurement just happened to have been made does not necessarily indicate the typical variation pattern of the relevant person. Thus, reproducibility of the blood pressure data obtained in the ABPM is becoming a problem in recent years. Specifically, the measurement of the blood pressure data was performed twice using the ABPM at different days and the results were compared, where many clinical researches such as non-patent document 1 have reported that the diagnosis result of the presence/absence of the nocturnal hypertension is covered at a percentage of 20 to 30%. In other words, the first problem is a problem in that an accurate blood variation pattern cannot be obtained with the ABPM which is loaned from the medical facility and can only be used for a limited number of days.

The second problem is a problem of tolerability of the patient himself/herself or the subject. During the use of the ABPM, the cuff constantly needs to be attached, and a box-shaped sphygmomanometer body needs to be carried around at the hip etc. The cuff and the sphygmomanometer body need to be connected with a tube for supplying air to the cuff. Furthermore, a band-shaped fixing tool generally needs to be attached to the shoulder to prevent the cuff from slipping in usual activities. If the blood pressure measurement is started up once in this state, the body activity and the conversation need to be interrupted for a few dozen seconds to obtain an accurate measurement. The subjects may feel unpleasant during summer, for example, as the interior of the cuff being constantly attached may become heated, or release abnormal odor. In particular, as the cuff of the ABPM is used by others, the unpleasant feeling is emphasized. The ABPM needs to be detached when taking a bath, but if the doctor determines that the attachment etc. of the cuff cannot be reliably performed, bathing may be prohibited. Due to the various reasons described above, the ABPM is said to be an examination in which the tolerability of the subject is low. Quite many patients reject the implementation although the examination is necessary.

The third problem is a problem in that the examination of low tolerability itself greatly influences the precision of blood pressure measurement. In other words, limitation of daily activities, attraction of public attention, and exposure to abnormal odor and uncleanliness become causes of psychological stress, and may lead to psychogenic blood pressure elevation. The blood pressure elevation effect is called novel effect, and is reported to continue for three to four days if the measurement with the ABPM is experimentally performed on successive days. That is, the true blood pressure may not be obtained with the normal ABPM examination that is performed only for one day or two days even from the standpoint of influencing the blood pressure itself.

As described above, the ABPM is considered as the only means capable of obtaining the blood pressure variation at the present stage, but measurement of high reliability is difficult to perform using the ABPM.

In view of the problems described above, the present invention aims to provide a sphygmomanometer capable of integrating blood pressure data measured fragmentarily over plural days to obtain the blood pressure variation pattern without constantly attaching the device over a long period of time, a blood pressure measurement system, and a measurement data program product.

MEANS FOR SOLVING THE PROBLEMS

In order to achieve the above aim, according to an aspect of the present invention, a sphygmomanometer includes a blood pressure measurement unit for measuring blood pressure; a timing unit for providing time information including at least a time of a time point a blood pressure measurement is performed by the blood pressure measurement unit; a storage unit for storing a measurement value obtained as a result of the blood pressure measurement in association with the time information; and a blood pressure integrating unit for performing a process using the respectively corresponded time on a plurality of measurement values stored in the storage unit, and calculating a blood pressure variation of a specific period.

Preferably, the blood pressure integrating unit calculates a measurement value corresponded to a time in a period of time of at least one part of a day of the measurement values stored in the storage unit, the measurement value being a blood pressure average value or an average value of at least one part of the measurement values. The sphygmomanometer preferably further includes a time selecting unit for specifying time; and a display unit for displaying the blood pressure average value of a period of time corresponding to a specified time.

Preferably, the blood pressure integrating unit calculates a blood pressure estimation function for estimating a blood pressure value corresponding to consecutive times based on the measurement value and the time information associated with the measurement value using at least one part of the measurement values stored in the storage unit. The sphygmomanometer preferably further includes a time selecting unit for specifying time; and a display unit for displaying the blood pressure estimated value using the blood pressure estimation function of a period of time corresponding to a specified time.

Preferably, the sphygmomanometer further includes a measurement value distribution evaluating unit for evaluating a temporal distribution in the specific period of the measurement values stored in the storage unit; wherein the blood pressure integrating unit calculates a blood pressure variation when satisfying a condition indicating whether or not the temporal distribution of the measurement values is a uniform distribution in the measurement value distribution evaluating unit. The condition is preferably based on the number of measurement values contained in a predetermined period. The measurement value distribution evaluating unit preferably performs the evaluation using total number of measurement values stored in the storage unit. The measurement value distribution evaluating unit preferably performs the evaluation using the time information associated with the measurement value stored in the storage unit. The blood pressure integrating unit preferably calculates a blood pressure estimation function for estimating a blood pressure value corresponding to consecutive times based on the measurement value and the time information associated with the measurement value using at least one part of the measurement values stored in the storage unit; and the measurement value distribution evaluating unit preferably calculates a difference with a blood pressure estimated value of the same time provided by the blood pressure estimation function for each measurement value stored in the storage unit as an error, and performs the evaluation based on the error.

Preferably, the timing unit provides time information related to a day of the week; and the blood pressure integrating unit classifies the measurement values stored in the storage unit to at least one group based on the day of the week and calculates the blood pressure variation.

Preferably, the sphygmomanometer further includes a measurement time providing unit for providing a measurement time; and an informing unit for informing a user that a time to perform the blood pressure measurement is reached based on the measurement time.

Preferably, the sphygmomanometer further includes a measurement time providing unit for providing a measurement time; and a control unit for controlling so as to automatically start-up the blood pressure measurement when a time to perform the blood pressure measurement is reached based on the measurement time.

The measurement time providing unit preferably provides a first measurement time in a first measurement date and a second measurement time in a second measurement date, the first measurement time and the second measurement time being different times.

According to another aspect of the present invention, a blood pressure measurement system includes a sphygmomanometer and an information processing device, wherein the sphygmomanometer includes a blood pressure measurement unit for measuring blood pressure, a timing unit for providing time information including at least a time of a time point a blood pressure measurement is performed by the blood pressure measurement unit, and an output unit for outputting the measurement value obtained as a result of the blood pressure measurement to the information processing device in association with the time information; the information processing device includes an acquiring unit for acquiring the measurement value and the time information in correspondence to each other from the sphygmomanometer, and storing the same in a storage device, an extracting unit for extracting, when a time of one day is divided by N as processing time, each measurement value corresponded with time information including a time included in first to $N^{th}$ processing times from the storage device, a calculating unit for calculating an average of the measurement values extracted in the extracting step for each of the first to the $N^{th}$ processing times, and a display unit for displaying an average of the measurement values for the first to the $N^{th}$ processing times.

The output unit of the sphygmomanometer and the acquiring unit of the information processing device preferably communicate to transmit and receive the measurement value and the time information. Preferably, the output unit of the sphygmomanometer outputs the measurement value and the time information to the information processing device through a recording medium; and the acquiring unit of the information processing device reads out the recording medium and acquires the measurement value and the time information.

According to another further aspect of the present invention, a measurement data process program relates to a computer readable program product storing a program for causing a computer to execute a process on a measurement value obtained as a result of a blood pressure measurement using a sphygmomanometer; the program causing the computer to execute a step of acquiring the measurement value and time information including at least a time of a time point the blood pressure measurement is performed in correspondence to each other from the sphygmomanometer and storing the same in a storage device; a step of extracting, when a time of one day is divided by N as processing time, each measurement value corresponded with time information including a time included in first to $N^{th}$ processing times from the storage device; a step of calculating an average of the measurement value extracted in the extracting step for the first to $N^{th}$ processing times; and a step of displaying an average of the measurement values for the first to $N^{th}$ processing times.

According to another further aspect of the present invention, a measurement data process program product relates to a computer readable program product storing a program for causing a computer to execute a process on a measurement value obtained as a result of a blood pressure measurement using a sphygmomanometer, the program causing the computer to execute a step of acquiring the measurement value and time information including at least a time of a time point the blood pressure measurement is performed in correspondence to each other from the sphygmomanometer; a step of determining a coefficient when representing a blood pressure value with a multidimensional formula having time as a variable using correspondence of the measurement value and the time information; and a step of displaying a curve represented by the multidimensional formula as a blood pressure variation cu rve.

Preferably, the program further causes the computer to execute steps of estimating a blood pressure value at a specified time.

The sphygmomanometer according to the present invention corresponds a measurement value and time information including at least a time of a time point the blood pressure measurement is performed, and calculates a blood pressure variation of a specific period by performing a process using such time, so that blood pressure data measured fragmentarily over a plurality of days can be integrated, and a blood pressure variation pattern can be obtained without constantly attaching the device over a long period of time.

BRIEF DESCRIPTION OF THE DRAWINGS

Fig. 1 is a view showing a specific example of an equipment configuration of a sphygmomanometer 1.

Fig. 2 is a flowchart showing a specific example of a flow of operations of the sphygmomanometer 1.
Fig. 3 is a flowchart showing a measurement/storage process.
Fig. 4 is a view showing a specific example of a measurement result stored in a blood pressure storage unit 17.
Fig. 5 is a flowchart showing a process of displaying a blood pressure average value for every period of time.
Fig. 6 is a view showing a specific example of a blood pressure variation display or the average value for every period of time.
Fig. 7A is a view showing a specific example of a display for specifying time.
Fig. 7B is a view showing a specific example of a display of the average value at a specified time.
Fig. 8 is a flowchart showing a blood pressure variation curve display process.
Fig. 9 is a flowchart showing an evaluation process of the time distribution.
Fig. 10 is a flowchart showing a process of calculating a residual.
Fig. 11 is a view showing a specific example of the blood pressure variation curve display.
Fig. 12A is a view showing a specific example of the display for specifying time.
Fig. 12B is a view showing a specific example of the display of an estimated value at the specified time.
Fig. 13 is a view showing a specific example of an equipment configuration of the sphygmomanometer 1 according to a first variant.
Fig. 14 is a view showing a specific example of a configuration of a system including the sphygmomanometer 1 according to a third variant.

## DESCRIPTION OF SYMBOLS

| | |
|---|---|
| 1 | sphygmomanometer |
| 5 | PC (Personal Computer) |
| 10 | housing |
| 11 | CPU |
| 12 | display unit |
| 13 | measurement start switch |
| 14 | blood pressure variation display switch |
| 15 | time input switch |
| 16 | blood pressure measurement unit |
| 17,55 | blood pressure storage unit |
| 18 | timing unit |
| 19 | measurement time providing unit |
| 20 | informing unit |
| 22 | transmitting unit |
| 30 | cuff |
| 50 | air tube |
| 51 | CPU |
| 52 | display unit |
| 53 | instruction input unit |
| 54 | receiving unit |

## BEST MODE FOR CARRYING OUT THE INVENTION

Embodiments of the present invention will be hereinafter described with reference to the drawings. Same reference numerals are denoted for the same parts and components in the following description. The names and the functions thereof are also the same.

A sphygmomanometer 1 is configured including a housing 10 incorporating a device for performing a process for blood pressure measurement and a cuff 30 to be attached to a subject, which are connected to an air tube 50, with reference to Fig. 1.

The housing 10 incorporates a CPU (central Processing Unit) 11, a blood pressure measurement unit 16, a blood pressure storage unit 17, and a timing unit 18. A display unit 12, a measurement start switch 13, a blood pressure variation display switch 14, and a time input switch 15 are arranged on an external surface of the housing 10.

The measurement start switch 13 is used for the operation to start the blood pressure measurement. The blood pressure variation display switch 14 is used for the operation to display blood pressure average value for every period of time, and blood pressure variation in blood pressure variation curve etc. The time input switch 15 is used when the user instructs a desired period of time or a desired time when displaying the display blood pressure average value for every period of time, and the blood pressure variation in blood pressure variation curve etc.

The CPU 11 is connected to the switches 13 to 15, and receives operation signals generated when the switches are operated. A program stored in the incorporated memory etc. is read out in response to the operation signal, and executed in the CPU 11.

In addition to the switches 13 to 15, the CPU 11 is connected to the display unit 12, the blood pressure measurement unit 16, and the blood pressure storage unit 17. Necessary controls signals are output thereto according to the execution of the program.

When receiving the operation signal instructing measurement start from the measurement start switch 13, the CPU 11 outputs the control signal to start-up the blood pressure measurement operation to the blood pressure measurement unit 16. The blood pressure measurement unit 16 measures the pressure of the cuff 30 through the air tube 50 according to the control signal, and measures the blood pressure of the subject. The blood measurement method of various principles are adopted for the blood pressure measurement methods in the blood pressure measurement unit 16. All of which are generally widely known methods, and the blood pressure measurement method adopted in the present invention is not limited to a specific method. The obtained measurement value is transmitted from the blood pressure measurement unit 16 to the CPU 11. The blood pressure measurement unit 16 is connected to the blood pressure storage unit 17, so that the obtained measurement value is stored in the blood pressure storage unit 17. The blood pressure storage unit 17 is also connected to the timing unit 18, thereby receiving time information related to measurement such as time and day of the week and storing the same in association with the measurement value.

When receiving the operation signal instructing the display of blood pressure average value of every time zone and blood pressure variation in the blood pressure variation curve and the operation signal indicating the desired period of time or the time from the blood pressure variation display switch 14 and the time input switch 15, the CPU 11 accesses the blood pressure storage unit 17, acquires a plurality of stored measurement values, and calculates the blood pressure variation value using the same.

The obtained blood pressure measurement value or the calculated blood pressure variation value in the CPU 11 are output to the display unit 12 along with the control signal for displaying.

The components of the sphygmomanometer 1 include, in addition to the components shown in Fig. 1, power supply device, power supply switch, and the like necessary for actually configuring the sphygmomanometer. Such components are components having generally widely known functions, and are not limited to a specific configuration in the present invention.

The processes of the sphygmomanometer 1 shown in the flowchart of Fig. 2 are realized when the CPU 11 reads out and executes the program stored in the incorporating memory etc. in response to the operation signals from the switches 13 to 15.

With reference to Fig. 2, in the sphygmomanometer 1, when detected that the measurement start switch 13 is pushed in the CPU 11 (YES in step S11), a measurement/storage process is executed (step S12). When detected that the blood pressure variation display switch 14 is pushed (YES in step S13), a blood pressure variation display process is executed (step S14), and when detected that a time is specified in the time input switch 15 (YES in step S15), a process of displaying the blood pressure value of the specified time is executed (step S16).

Each process of steps S12, 14 will be described below.

[Measurement/storage process]

With reference to Fig. 3, when detected that the measurement start switch 13 is pushed in the CPU 11 in step S11, the blood pressure measurement is executed in the blood pressure measurement unit 16 in step (hereinafter abbreviated as ST.) 102. A method generally widely known as described above is adopted for the blood pressure measurement method herein. The CPU 11 stores the measurement values such as systolic blood pressure, diastolic blood pressure, and pulse rate, and the time information in correspondence to each other in the blood pressure storage unit 17 in ST. 103, 104, and terminates the series of operations.

With reference to Fig. 4, the blood pressure storage unit 17 stores the measurement unit such as systolic blood pressure (SBP), diastolic blood pressure (DBP), and pulse rate (PR) and the time information such as day of the week and time in correspondence to each other as measurement results.

The time information stored in correspondence to the measurement value merely need to include the day of the week and the time, at the very least, for the time information used in the process of the present invention. In the process of the present invention to be hereinafter described, only the time and/or day of the week is used and the date is not used for the identification of data stored in the blood pressure storage unit 17. According to such correspondence, the data measured in different days can be integrated using the time and/or day of the week. Obviously, the information about the date can be stored as time information, and plurality of data may be integrated while limiting the number of days and the period, or use in other processes is also considered, but assumption is made that only the day of the week and the time are included as the time information for the sake of simplifying the explanation.

[Blood pressure average value display process]

A process of displaying the blood pressure average value and the estimated value for every period of time will be described as the blood pressure variation display process of step S14.

Fig. 5 is a flowchart showing a process of displaying the blood pressure average value for every period of time. The following description will be made with the period of time set at an interval of every one hour (i.e., unit of what hour), but a few hours may be set as one period of time. Furthermore, the description of the flow of the process below is made on the systolic blood pressure, but the process is the same for the diastolic blood pressure and the pulse rate.

With reference to Fig. 5, first the CPU 11 initializes a pointer h of the period of time to zero in ST. 101, and initializes a pointer i of the data stored in the blood pressure storage unit 17, a counter n of the number of data used in calculating the average value, and a variable Σ(h) holding the sum of the data used in calculating the average value all to zero in ST. 102.

Whether or not time information T(i) corresponding to the data specified with the data pointer i is within a target range of the average value calculation is determined in ST. 103. Since the period of time is set to an interval of every one hour, within the target range will be between 0:00 to 0:59 (in case of units in minutes) if h=0. If determined that the time information T(i) is within the range (YES in ST. 103), the CPU 11 adds the measurement value SBP(i) to the variable Σ(h) in ST. 104, and adds one to the counter n in ST. 105.

In ST. 106, one is added to the data pointer i, and whether or not the data pointer i has exceeded the stored number of data iMAX is determined in ST. 107. If i≤iMAX (NO in ST. 107), the process returns to ST. 103, and the same process is repeated on the next data.

If i>iMAX (YES in ST. 107), the sum Σ(h) of all the data that satisfy the condition of the data in the blood pressure storage unit 17, and the number of data n thereof are determined, and an average value ASBP(h)= Σ(h)/n is calculated in ST. 108. Thereafter, one is added to the pointer h of the period of time in ST. 109, and whether or not the time information T(i) exceeds the last time hand (h=24) is determined in ST. 110. If determined as not exceeding (NO in ST. 110), the process returns to ST. 102, and similar process is repeated on the next period of time. If determined as exceeding (YES in ST. 110), a process for displaying the average value on the display unit 12 is executed in ST. 111, and the series of processes are terminated.

Thus, the blood pressure measurement values measured at various periods of times are integrated over a plurality of days with the interval for every predetermined time width as a reference, and calculated as the blood pressure variation of one day.

Fig. 6 is a view showing a specific example of the blood pressure variation display or the average value for every period of time of an interval of every one hour displayed on the display unit 12 as a result of the process in ST. 111. As shown in Fig. 6, in ST. 111, a process of connecting the average values for every period of time obtained through the above processes with a line segment, integrating the blood pressure measurement values over a plurality of days, and displaying a graph showing the blood pressure variation of one day is executed. The display of the blood pressure variation serving as the average value may be in graph display as shown in Fig. 6, or the average values for every period of time may be displayed in a form of a list.

If a time is specified using the time input switch 15 with the blood pressure variation displayed as shown in Fig. 6 (YES in step S15), the CPU 11 acquires and displays the average value of the specified time or the average value of the period of time including the specified time from the average values for every period of time obtained as a result of the above-described processes. Alternatively, an estimated value of the specified time is calculated from the average values for every period of time obtained as a result of the above-described processes, and displayed. The calculation method here simply includes a method of obtaining using a linear function of the average values of two period of times sandwiching the specified time.

Fig. 7A is a view showing a specific example of the display for specifying the time while the blood pressure variation is being displayed as shown in Fig. 6. In this case, the time input switch 15 includes a ten key etc., and the time is input specifically using the ten key. Fig. 7B is a view showing a specific example of the display of the average value at the specified time. As shown in Fig. 7B, to which part of the blood pressure variation of the day the specified time corresponds is preferably displayed in addition to the display of the average value.

The method of specifying the time and the displaying method are not limited to the methods shown in Fig. 7A and Fig. 7B, and all other methods may be adopted. For instance, the time input switch 15 may include left and right keys and a scroll key, and a mark indicating a certain time may be included in the blood pressure variation display (thick line in Fig. 7B), so that the left and right keys and the scroll key are operated to move the mark on the blood pressure variation display so as to reach the desired time, thereby displaying the average value of the time where the mark is located.

The timing of specifying the time is not limited to the timing of displaying the blood pressure variation, and the time may be specified regardless of the display of the blood pressure variation. In such case, the average value of the specified time, the average value of the period of time including the specified time, or the estimated value of the specified time may be displayed without carrying out the blood pressure variation display. This is the same in the following processes.

[Display process of blood pressure variation curve]

As the blood pressure variation display process in step S14, a process of displaying the blood pressure variation curve or the change curve of the blood pressure defined on the time (from 0 o'clock to immediately before 0 o'clock the next day) axis of one day will be described. The blood pressure variation curve shows at what time and how much the blood pressure of the subject went up/went down.

First, the blood pressure variation curve is a function of time t (e.g., time in minutes with 0 AM as the reference), and is defined with the following general expression.

$$BP(t)=At^3+Bt^2+Ct+D \qquad \text{Eq. (1)}$$

Here, constants A, B, C, and D are determined by a plurality of blood pressure values and the corresponding time stored in the blood pressure storage unit 17. More specifically, the above constants are determined by having the equation (1) to a special equation as an approximate expression of the blood pressure value and the time. This is a general numerical analysis method.

Fig. 8 is a flowchart showing the display process of the blood variation curve. With reference to Fig. 8, a function F(t) of the blood pressure variation curve is first specified in ST. 201. In other words, the CPU 11 specifies the constants A, B, C, and D of equation (1), and obtains a function of a specific time. Widely known methods such as least square method and entropy method are known for the specific method, all of which methods can be used.

The CPU 11 evaluates time distribution in ST. 202. Here, the blood pressure variation curve is assumed to be defined in a time zone of twenty-four hours (defined zone). The length of the defined zone may be changed according to the purpose of the process and the collecting manner of data, but in either case, the measurement value data needs to be evenly distributed to a certain extent over the entire defined zone in order to accurately calculate the blood pressure variation curve. Determining the condition thereof is the evaluation of the time distribution.

In ST. 203, the value of the variable TV indicating the evaluation result of the time distribution performed in ST. 202 is referenced, where the process proceeds to ST. 204 if the evaluation result shows 1 indicating that the condition for calculating the blood pressure variation curve is satisfied (YES in ST. 203), or the process proceeds to ST 207 if not (NO in ST. 203) and displays an error display notifying that the blood pressure variation curve cannot be calculated and terminates the process.

In ST. 204, a residual E is calculated in the CPU 11 in order to evaluate to what extent the blood pressure variation curve determined in ST. 201 matches the actually measured measurement value. The residual is one numerical value representing the difference between the plurality of actually measured values (measurement values) and the blood pressure variation curve.

In ST. 205, whether or not the residual E is greater than a threshold value TH indicating an allowable range is determined, where the process proceeds to ST. 207 assuming the accurate blood pressure variation curve cannot be obtained if greater (YES in ST. 205), which is notified through error display, and the series of processes are terminated.

If the residual E is within the threshold value TH (NO in ST. 205), the blood pressure variation curve is determined as sufficiently and accurately representing the measurement values, and the CPU 11 executes a process of displaying the blood pressure variation curve on the display unit 12 in ST. 206, and terminates the series of processes.

Fig. 9 is a flowchart showing an evaluation process of the time distribution executed in ST. 202. First, in this process, the defined zone is divided into plural regions. For example, the defined zone of twenty-four hours is divided into four sub-zones each having a length if six hours.

With reference to Fig. 9, the CPU 11 first initializes the pointer h of the sub-zone to one, and the variable TV representing the evaluation result to zero in ST. 301. In ST. 302, the pointer i of the measurement value data is initialized to one, and the counter N(h) representing the number of measurement value data contained in the sub-zone to zero.

In ST. 303, whether or not the time information T(i) corresponded to the measurement value specified with the pointer i is within the range of the sub-zone specified with the pointer h, where the counter (h) is added with one if within the range (YES in ST. 303).

One is added to the pointer i in ST. 305, and whether or not the pointer i exceeded the variable iMAX representing the total number of data is determined in ST. 306. If not exceeded (NO in ST. 306), the process returns to ST. 303, and the same process is repeated for the next data.

If the pointer i exceeded the variable iMAX in ST. 306, that is, if i>iMAX (YES in ST. 306), the process proceeds to ST. 307, and whether or not the number of data N(h) contained in the range of the sub-zone specified with the pointer h is greater than or equal to five is determined. If the number of data N(h) is smaller than five (NO in ST. 307), the process is terminated assuming the number of data is lacking. If the number of data N(h) is greater than or equal to five, that is, if $N(h) \geq 5$ (YES in ST. 307), one is added to the pointer h in ST. 308-1, and whether or not the pointer h exceeded the number hMAX of sub-zones is determined in ST. 308-2. If not exceeded (NO in ST. 308-2), the process returns to ST. 302, and the same process is repeated for the next sub-zone.

If the pointer h exceeded the number hMAX of sub-zones, that is, if hMAX<h (YES in ST 308-2), the variation of the number of data contained in each sub-zone is evaluated in ST. 309. Various methods are contrived for methods of evaluating such variation, and may be a method of calculating a standard deviation SD of the number of data contained in each sub-zone, and determining whether or not it exceeds a threshold value SDMAX indicating the allowable range. A general standard deviation calculation method is adopted for the method of calculating the standard deviation SD. In ST. 309, the CPU 11 performs such determination, and if the standard deviation SD is within the allowable range, one is substituted to the variable TV representing the same, and the series of processes are completed.

Fig. 10 is a flowchart showing a process of calculating a residual executed in ST. 204. With reference to Fig. 10, the CPU 11 first initializes the pointer i of the data to one, and the sum variable Σ to zero in ST. 401.

In ST. 402, the square of the difference F (T(i))-SBP(i)of the value (T(i)) of the blood pressure variation curve at time T(i) corresponded to the measurement value specified with the pointer i and the measurement value SBP(i) is added to the sum variable Σ. One is added to the pointer i in ST. 403, whether or not the pointer i exceeded the total number of data iMAX is determined in ST. 404, and the process returns to ST. 402 and the same process is repeated if not exceeded (NO in ST. 404). If the pointer i exceeded the total number of data iMAX (YES in ST. 404), the process proceeds to ST. 405, and the residual E is calculated. The residual E is obtained by dividing the sum of squares of the difference of the blood pressure variation curve substituted to the sum variable Σ and all measurement values with the number of data iMAX.

Fig. 11 is a view showing a specific example of the blood pressure variation curve display displayed on the display unit 12 as a result of the above processes. In order to obtain such blood pressure variation curve, the ABPM had to be always attached in the prior art, but as the above-described processes can be executed with the sphygmomanometer 1, the blood pressure variation curve of one day can be calculated by integrating the blood pressure values measured in different period of times, in some days, over a plurality of days.

If the time is specified using the time input switch 15 while the blood pressure variation is being displayed as shown in Fig. 11 (YES in step S15), the CPU 11 acquires the value of the specified time from the blood pressure variation curve obtained as a result of the above processes, and displays the same as the estimated value of the specified time.

Fig. 12A is a view showing a specific example of the display for specifying the time while the blood pressure variation is being displayed as shown in Fig. 11. In this case, the time input switch 15 includes a ten key etc., and the time is input specifically using the ten key. Fig. 12B is a view showing a specific example of the display of the estimated value at the specified time. As shown in Fig. 12B, to which part of the blood pressure variation of the day the specified time corresponds is preferably displayed in addition to the display of the estimated value.

[Variant 1]

In the above described embodiment, assuming the blood pressure measurement starts when pushing of the measurement start switch 13 is detected in step S11, the blood pressure measurement can be performed at an arbitrary time of the user, but the measurement time may be informed to the user at the preset time, or the blood pressure measurement may be automatically started when the preset time is reached. The manual start and the automatic start may be combined.

With reference to Fig. 13, the sphygmomanometer 1 according to a first variant includes a measurement time providing unit 19 and an informing unit 20, in addition to the configuration shown in Fig. 1. The output of the timing unit 18 is also provided to the CPU 11.

The measurement time providing unit 19 stores the measurement time, and provides the information to the CPU 11. The measurement time may be stored in advance, or may be input and stored using a predetermined operation means (e.g., simultaneous use of time input switch 15, or operation means (not shown) may be arranged).

The CPU 11 continues to constantly receive the current time from the timing unit 18, and compares the current time and the next measurement time provided from the measurement time providing unit 19. At the point the times match, a control signal is output to the informing unit 20. The informing unit 20 informs the user that it is the measurement time in response to the control signal from the CPU 11. The informing method is not limited, and various informing methods such as voice and vibration may be adopted.

Furthermore, apart from when the control signal is output to the informing unit 20, the CPU 11 outputs the control signal to the blood pressure measurement unit 16 at the point the current time and the next measurement time match, and the blood pressure measurement may be automatically started. In this case, the informing unit 20 itself may not be included in the sphygmomanometer 1. The blood pressure measurement may be automatically started after the control signal is output to the informing unit 20, and the informing unit 20 informs the user that it is the measurement time.

Furthermore, the measurement time providing unit 19 may provide measurement time that differs depending on the day to the CPU 11, or the measurement time providing unit 19 may interiorly include a means for randomly generating time using a random function etc. to provide the measurement time so that measurement is performed at a random time or the measurement time may be provided while shifting the period of time for executing the blood pressure measurement by every predetermined number of days. Thus, a result without bias at the uniformly distributed time can be obtained when obtaining the blood pressure average value for every period of time by integrating the blood pressure data for a plurality of days through the above processes, or calculating the blood pressure variation curve, and the precision of the blood pressure variation of one day to be calculated can be enhanced.

Since the user can acquire the blood pressure variation data of one day by simply repeating the blood pressure measurement over a few number of times in one day, the load of the user is reduced. In particular, when measuring the blood pressure even while sleeping, measurement had to be performed over and over (e.g., every one hour) in the prior art, and thus the user wakes up every time and cannot get enough sleep, and the load of the user was large. In the sphygmomanometer according to the present embodiment, however, the number of measurements per one day can be reduced by dispersing the measuring time during sleep for every day, and sleep disorder can be reduced. For instance, the measurement is set to be performed at 23 o'clock, 2 o'clock, and 5 o'clock at night on the first day. The measurement is set to be performed at 0 o'clock, 3 o'clock, and 6 o'clock at night on the second day. The measurement is set to be performed at 1 o'clock, 4 o'clock, and 7 o'clock at night on the third day. If measured according to such setting, the measurement is performed only three times during sleep in one day, and the measurement value during sleep for every one hour is obtained after integration.

[Variant 2]

In the above process, the blood pressure variation display representing the blood pressure variation of one day is performed, but variation may be calculated while dividing into groups based on the day of the week.

According to some of the recent clinical researches, the blood pressure varies within one week (weekly variation), and it is considered medically significant to observe such variation. For instance, this is represented by the fact that the blood pressure of the worker who takes days off on weekends differs between weekday and weekend. It is clinically useful if the above described blood pressure average value and the blood pressure estimated curve can be compared between weekday and weekend.

This is specifically realized by acquiring data necessary for calculating using the data DW(i) indicating the day of the week of the stored data shown in Fig. 4, and performing the calculation of the blood pressure average value and the blood pressure estimated curve separately. That is, in the process shown in Fig. 5, whether or not the information DW(i) indicating the day of the week is a predetermined value (i.e., whether or not extracted measurement value is the measurement value of the predetermined day of the week) is determined after initialization in ST. 102, and the processes after ST. 103 is executed if at the predetermined value.

[Variant 3]

In the above description, the processes are all assumed to be executed in the CPU 11 of the sphygmomanometer 1, but the sphygmomanometer 1 may include a means for outputting the measurement result to an external computer device separate from the sphygmomanometer 1 such as personal computer (hereinafter referred to as PC) and all or one part of the process may be performed in the relevant device.

Fig. 14 is a view showing a specific example of a configuration of a system including the sphygmomanometer 1, and specifically, is a view showing a specific example of a configuration of the sphygmomanometer 1 and the PC 5. The sphygmomanometer 1 and the PC 5 are connected in a wired or a wireless manner.

With reference to Fig. 14, the sphygmomanometer 1 according to the third variant is configured including a transmitting unit 22 for outputting the measurement result to another device, in addition to the configuration shown in Fig. 1. If the blood pressure variation display process is executed in another device, the blood pressure variation display switch 14 and the time input switch 15 for performing the relevant process do not need to be included.

The transmitting unit 22 transmits the data of the measurement result stored in the blood pressure storage unit 17 as shown in Fig. 4 to the PC 5 in response to the control signal from the CPU 11.

The PC 5 is configured including a CPU 51 for entire control, a display unit 52 such as display, an instruction input unit 53 such as keyboard and mouse, a receiving unit 54 for receiving the measurement result transmitted from the transmitting unit 22, and a blood pressure storage unit 55 for storing the received measurement result.

The sphygmomanometer 1 may include a write unit for writing the measurement result to a recording medium in place of the transmitting unit 22, and the PC 5 may include a read-out unit for reading out the measurement result from the recording medium in place of the receiving unit 54, so that the sphygmomanometer 1 and the PC 5 exchange the measurement result via the recording medium.

In the third variant, when instruction to display the blood pressure variation or specification of time to display the blood pressure value or the estimated value is accepted at the input unit 53 of the PC 5, the measurement result received at the receiving unit 54 and stored in the blood pressure storage unit 55 is acquired, and the above-described process is executed in the CPU 51.

A program for executing the above processes in the PC 5 may be provided. Such program can be recorded in a computer readable recording medium such as a flexible disc, CD-ROM (Compact Disc-Read Only Memory), ROM (Read Only Memory), RAM (Random Access Memory), memory card, and the like attached to the computer, and provided as a program product. Alternatively, the program can be provided by being recorded in a recording medium such as hard disc embedded in the computer. The program can be provided by being downloaded via the network.

The program according to the present invention may call out the necessary module at a predetermined array and a predetermined timing from the program modules provided as one part of the operating system (OS) of the computer to execute the process. In this case, the module is not included in the program itself, and the process is executed in cooperation with the OS. The program that does not include such module is also encompassed in the program according to the present invention.

The program according to the present invention may be provided by being incorporated in one part of another program. In this case as well, the module included in another program is not included in the program itself, and the process is executed in cooperation with the other program. The program incorporated in another program is also encompassed in the program according to the present invention.

The provided program product is installed in a program storage unit such as hard disc, and then executed. The program product includes the program itself, and the recording medium recorded with the program.

The embodiments disclosed herein are merely illustrative in all aspects and should not be construed as being restrictive. The scope of the invention is defined by the appended claims rather than by the description preceding them, and all changes that fall within meets and bounds of the claims, or equivalence of such meets and bounds are therefore intended to be embraced by the claims.

## Claims

**1.** A sphygmomanometer comprising:

a blood pressure measurement unit (16) for measuring blood pressure;
a timing unit (18) for providing time information including at least a time of a time point a blood pressure measurement is performed by the blood pressure measurement unit;
a storage unit (17) for storing a measurement value obtained as a result of the blood pressure measurement in association with the time information; and
a blood pressure integrating unit (11) for performing a process using the respectively corresponded time on a plurality of measurement values stored in the storage unit, and calculating a blood pressure variation of a specific period.

**2.** The sphygmomanometer according to claim 1, wherein the blood pressure integrating unit calculates a measurement value corresponded to a time in a period of time of at least one part of a day of the measurement values stored in the storage unit, the measurement value being a blood pressure average value or an average value of at least one part of the measurement values.

**3.** The sphygmomanometer according to claim 2, further comprising:

a time selecting unit (15) for specifying time; and
a display unit (12) for displaying the blood pressure average value of a period of time corresponding to a specified time.

**4.** The sphygmomanometer according to claim 1, wherein the blood pressure integrating unit calculates a blood pressure estimation function for estimating a blood pressure value corresponding to consecutive times based on the measurement value and the time information associated with the measurement value using at least one part of the measurement values stored in the storage unit.

**5.** The sphygmomanometer according to claim 4, further comprising:

a time selecting unit (15) for specifying time; and
a display unit (12) for displaying the blood pressure estimated value using the blood pressure estimation function of a period of time corresponding to a specified time.

**6.** The sphygmomanometer according to claim 1, further comprising:

a measurement value distribution evaluating unit (11) for evaluating a temporal distribution in the specific period of the measurement values stored in the storage unit; wherein
the blood pressure integrating unit calculates a blood pressure variation when satisfying a condition indicating whether or not the temporal distribution of the measurement values is a uniform distribution in the measurement value distribution evaluating unit.

**7.** The sphygmomanometer according to claim 6, wherein the condition is based on the number of measurement values contained in a predetermined period.

**8.** The sphygmomanometer according to claim 6, wherein the measurement value distribution evaluating unit performs the evaluation using total number of measurement values stored in the storage unit.

**9.** The sphygmomanometer according to claim 6, wherein the measurement value distribution evaluating unit performs the evaluation using the time information associated with the measurement value stored in the storage unit.

**10.** The sphygmomanometer according to claim 6, wherein
the blood pressure integrating unit calculates a blood pressure estimation function for estimating a blood pressure value corresponding to consecutive times based on the measurement value and the time information associated with the measurement value using at least one part of the measurement values stored in the storage unit; and
the measurement value distribution evaluating unit calculates a difference with a blood pressure estimated value of the same time provided by the blood pressure estimation function for each measurement value stored in the storage unit as an error, and performs the evaluation based on the error.

**11.** The sphygmomanometer according to claim 1, wherein
the timing unit provides time information related to a day of the week; and
the blood pressure integrating unit classifies the measurement values stored in the storage unit to at least one group based on the day of the week and calculates the blood pressure variation.

**12.** The sphygmomanometer according to claim 1, further comprising:

a measurement time providing unit (19) for providing a measurement time; and
an informing unit (20) for informing a user that a time to perform the blood pressure measurement is reached based on the measurement time.

**13.** The sphygmomanometer according to claim 1, further comprising:

a measurement time providing unit (19) for providing a measurement time; and
a control unit (11) for controlling so as to automatically start-up the blood pressure measurement when a time to perform the blood pressure measurement is reached based on the measurement time.

**14.** The sphygmomanometer according to claim 12 or 13, wherein the measurement time providing unit provides a first measurement time in a first measurement date and a second measurement time in a second measurement date, the first measurement time and the second measurement time being different times.

**15.** A blood pressure measurement system comprising a sphygmomanometer (1) and an information processing device (5), wherein
the sphygmomanometer includes,
a blood pressure measurement unit (16) for measuring blood pressure,
a timing unit (18) for providing time information including at least a time of a time point a blood pressure measurement is performed by the blood pressure measurement unit, and
an output unit (22) for outputting the measurement value obtained as a result of the blood pressure measurement to the information processing device in association with the time information;

the information processing device includes,
an acquiring unit (54) for acquiring the measurement value and the time information in correspondence to each other from the sphygmomanometer, and storing the same in a storage device (55),
an extracting unit (51) for extracting, when a time of one day is divided by N as processing time, each measurement value corresponded with time information including a time included in first to $N^{th}$ processing times from the storage device,
a calculating unit (51) for calculating an average of the measurement values extracted in the extracting step for each of the first to the $N^{th}$ processing times, and
a display unit (52) for displaying an average of the measurement values for the first to the $N^{th}$ processing times.

**16.** The blood pressure measurement system according to claim 15, wherein the output unit of the sphygmomanometer and the acquiring unit of the information processing device communicate to transmit and receive the measurement value and the time information.

**17.** The blood pressure measurement system according to claim 15, wherein
the output unit of the sphygmomanometer outputs the measurement value and the time information to the information processing device through a recording medium; and
the acquiring unit of the information processing device reads out the recording medium and acquires the measurement value and the time information.

**18.** A computer readable measurement data process program product storing a program for causing a computer to execute a process on a measurement value obtained as a result of a blood pressure measurement using a sphygmomanometer; the program causing the computer to execute:

a step (S12) of acquiring the measurement value and time information including at least a time of a time point the blood pressure measurement is performed in correspondence to each other from the sphygmomanometer and storing the same in a storage device;
a step (ST. 108) of extracting, when a time of one day is divided by N as processing time, each measurement value corresponded with time information including a time included in first to $N^{th}$ processing times from the storage device;
a step (ST. 108) of calculating an average of the measurement value extracted in the extracting step for the first to $N^{th}$ processing times; and
a step (ST. 111) of displaying an average of the measurement values for the first to $N^{th}$ processing times.

**19.** A computer readable measurement data process program product storing a program for causing a computer to execute a process on a measurement value obtained as a result of a blood pressure measurement using a sphygmomanometer, the program causing the computer to execute:

a step (S12) of acquiring the measurement value and time information including at least a time of a time point the blood pressure measurement is performed in correspondence to each other from the sphygmomanometer;
a step (ST. 201) of determining a coefficient when representing a blood pressure value with a multidimensional formula having time as a variable using correspondence of the measurement value and the time information; and
a step (ST. 206) of displaying a curve represented by the multidimensional formula as a blood pressure variation curve.

**20.** The program product of measurement data process according to claim 18 or 19, wherein the program further causes the computer to execute steps (S14, S16) of estimating a blood pressure value at a specified time.

Fig. 1

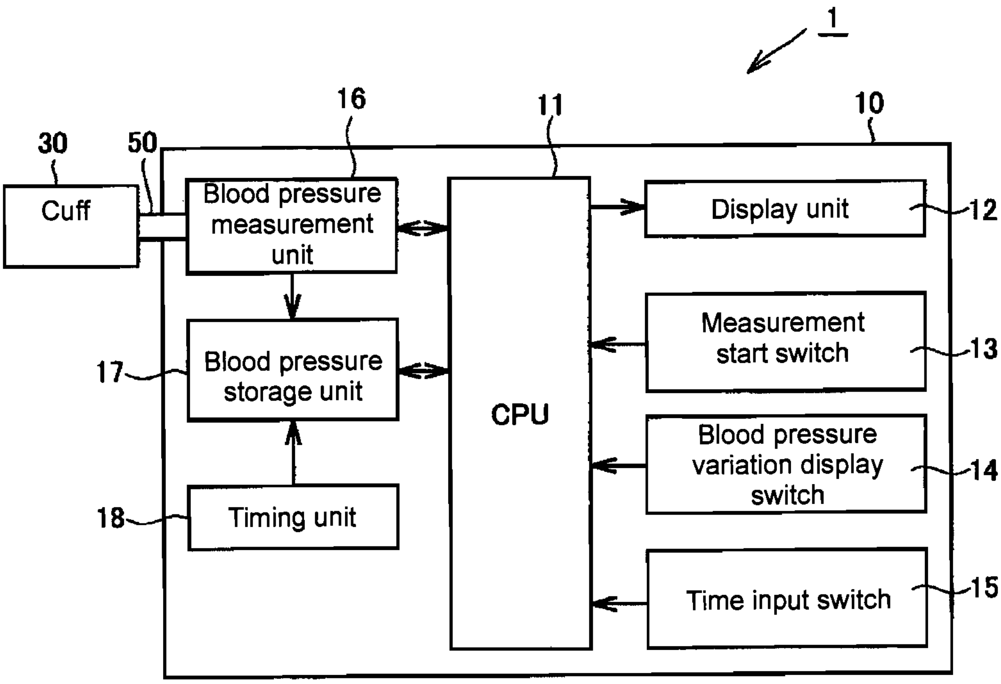
1
16
11
10
30
50
Cuff
Blood pressure measurement unit
Display unit
12
Measurement start switch
13
17
Blood pressure storage unit
CPU
Blood pressure variation display switch
14
18
Timing unit
Time input switch
15

Fig. 2

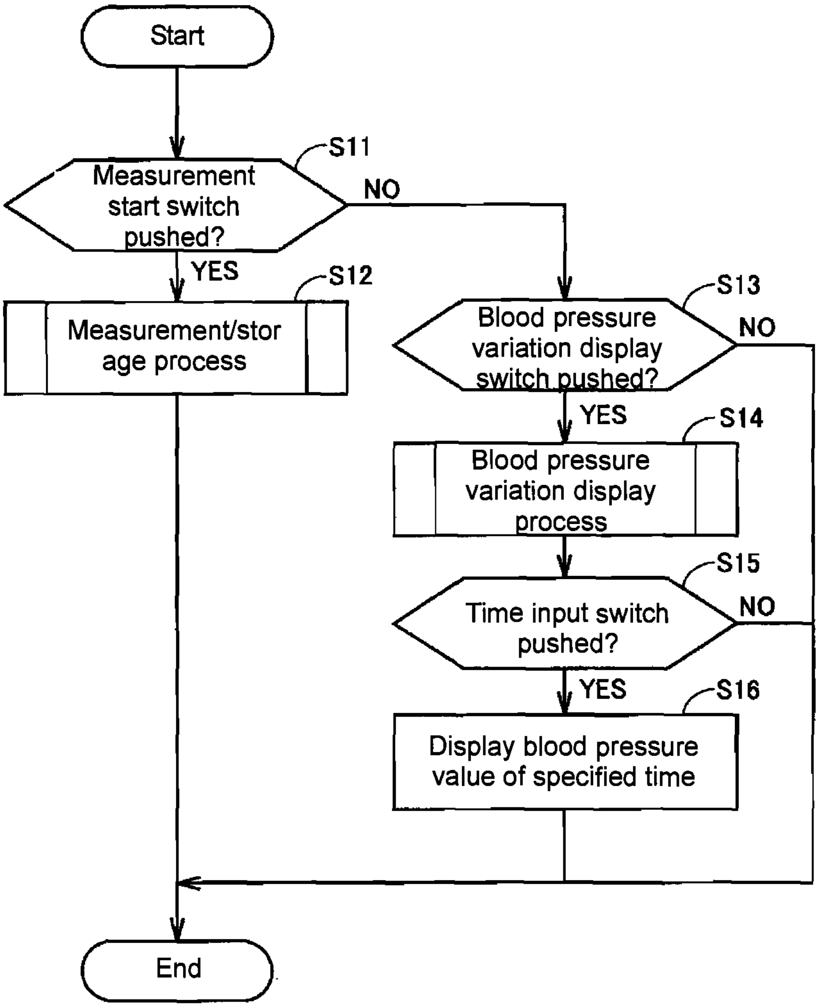
Start
S11
Measurement start switch pushed?
NO
YES
S12
Measurement/storage process
S13
Blood pressure variation display switch pushed?
NO
YES
S14
Blood pressure variation display process
S15
Time input switch pushed?
NO
YES
S16
Display blood pressure value of specified time
End

Fig. 3

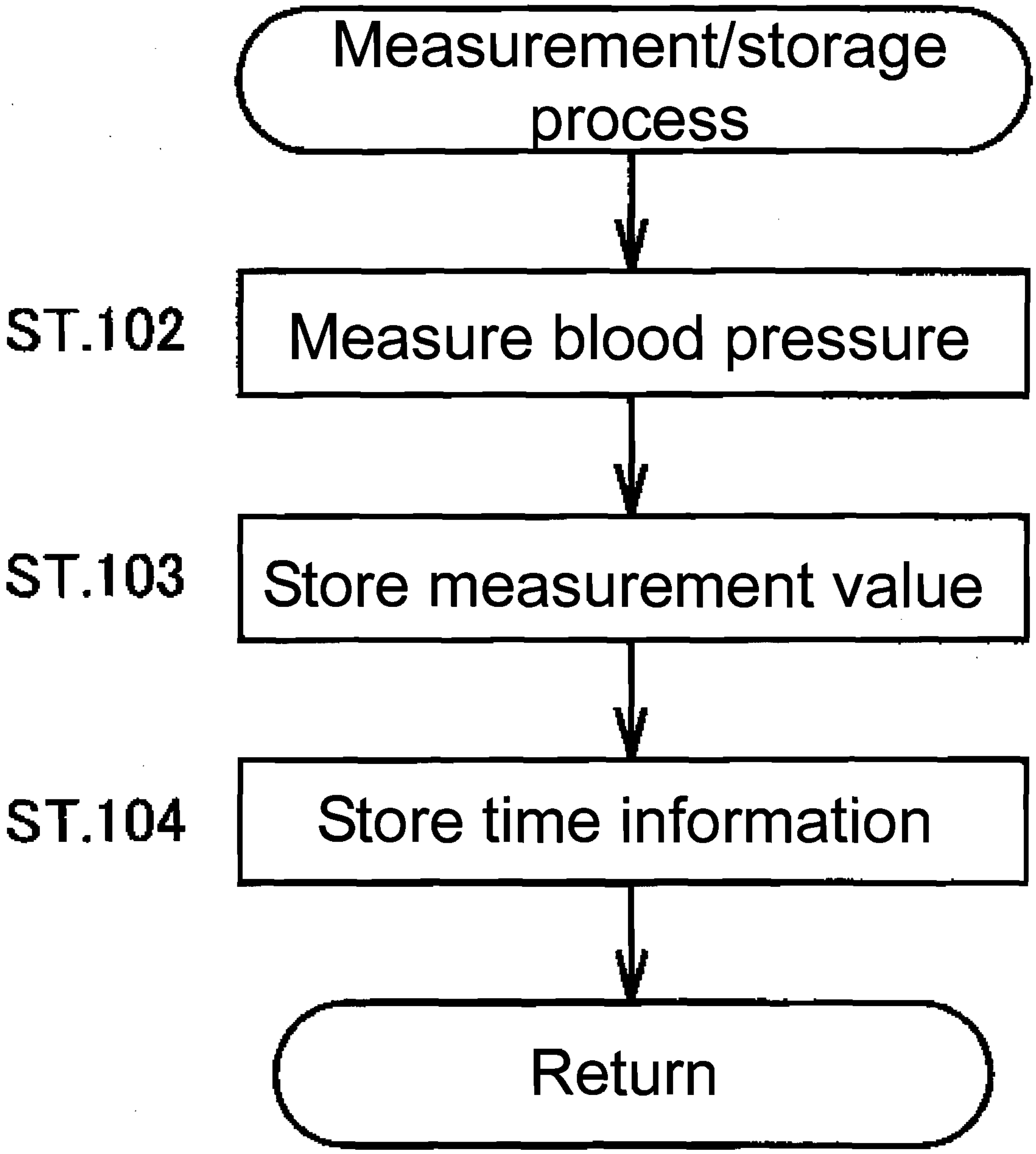
Measurement/storage process
ST.102
Measure blood pressure
ST.103
Store measurement value
ST.104
Store time information
Return

Fig. 4

| Systolic blood pressure | Diastolic blood pressure | Pulse rate | Day of week | Time |
|---|---|---|---|---|
| SBP(1) | DBP(1) | PR(1) | DW(1) | T(1) |
| SBP(2) | DBP(2) | PR(2) | DW(2) | T(2) |
| SBP(3) | DBP(3) | PR(3) | DW(3) | T(3) |
| SBP(4) | DBP(4) | PR(4) | DW(4) | T(4) |
| SBP(5) | DBP(5) | PR(5) | DW(5) | T(5) |
| : | : | : | : | : |
| | | | | |

Fig. 5

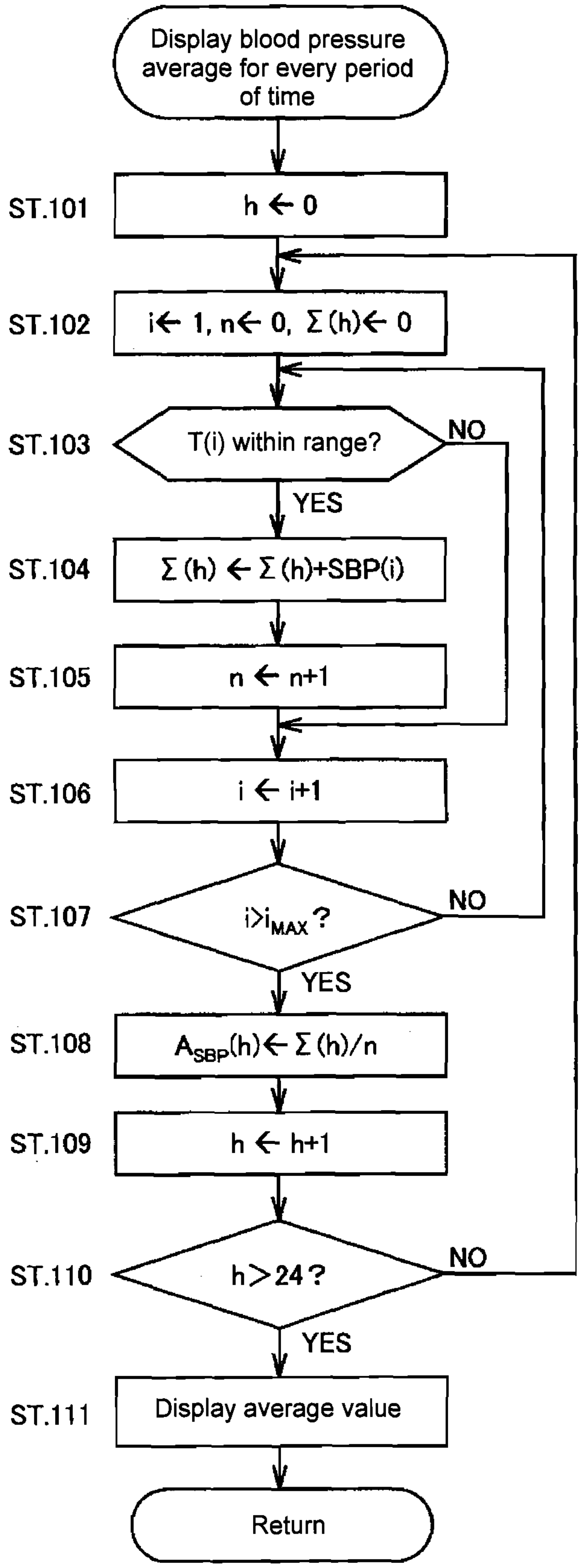
Display blood pressure average for every period of time
ST.101
h ← 0
ST.102
i← 1, n← 0, Σ(h)← 0
ST.103
T(i) within range?
NO
YES
ST.104
Σ(h) ← Σ(h)+SBP(i)
ST.105
n ← n+1
ST.106
i ← i+1
ST.107
i>i_MAX ?
NO
YES
ST.108
A_SBP(h)← Σ(h)/n
ST.109
h ← h+1
ST.110
h>24 ?
NO
YES
ST.111
Display average value
Return

Fig. 6

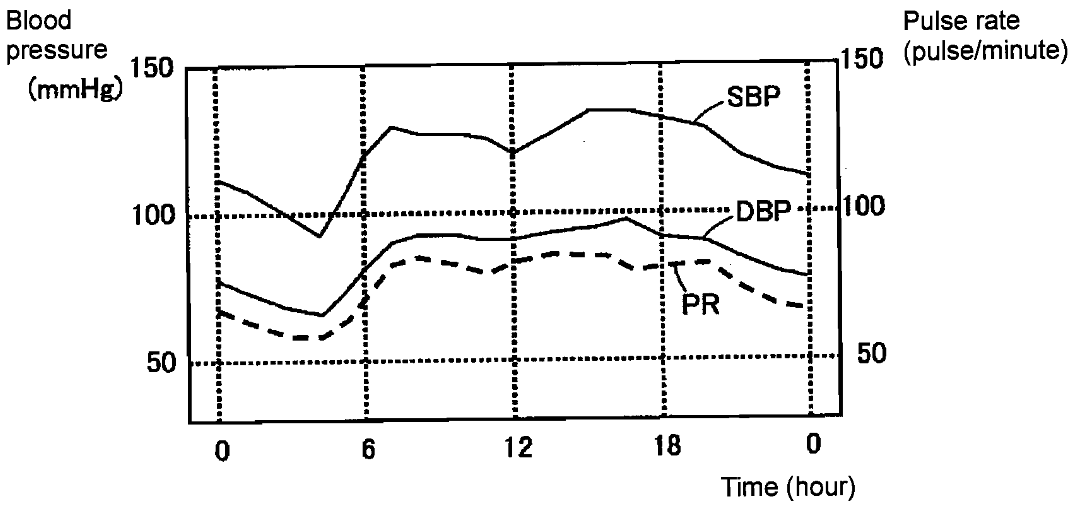
Blood pressure (mmHg)
150
100
50
Pulse rate (pulse/minute)
150
100
50
SBP
DBP
PR
0
6
12
18
0
Time (hour)

Fig. 7A

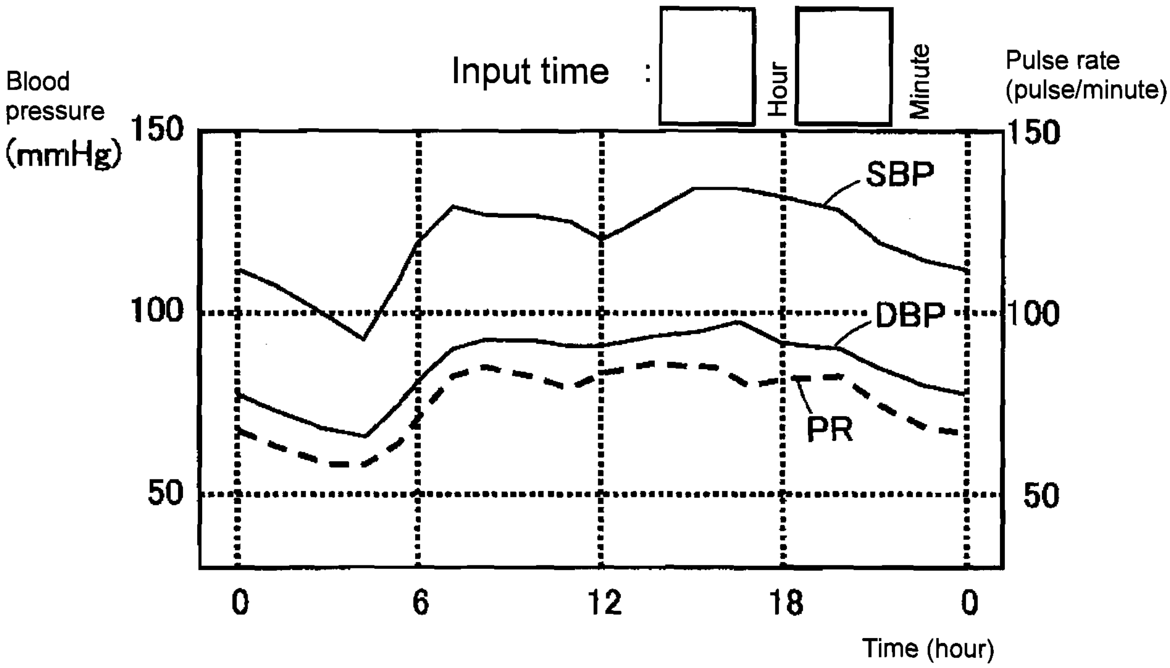
Input time :
Hour
Minute
Blood pressure (mmHg)
Pulse rate (pulse/minute)
150
100
50
SBP
DBP
PR
0
6
12
18
0
Time (hour)

Fig. 7B

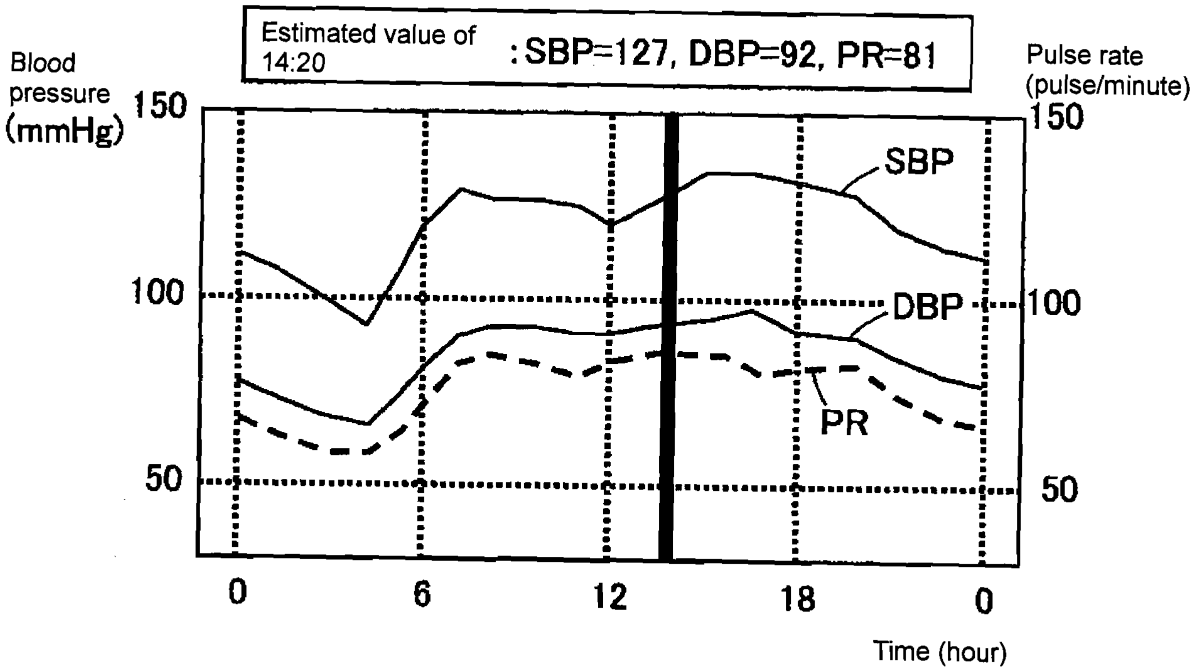
Estimated value of 14:20
: SBP=127, DBP=92, PR=81
Blood pressure (mmHg)
150
100
50
Pulse rate (pulse/minute)
150
100
50
SBP
DBP
PR
0
6
12
18
0
Time (hour)

Fig. 8

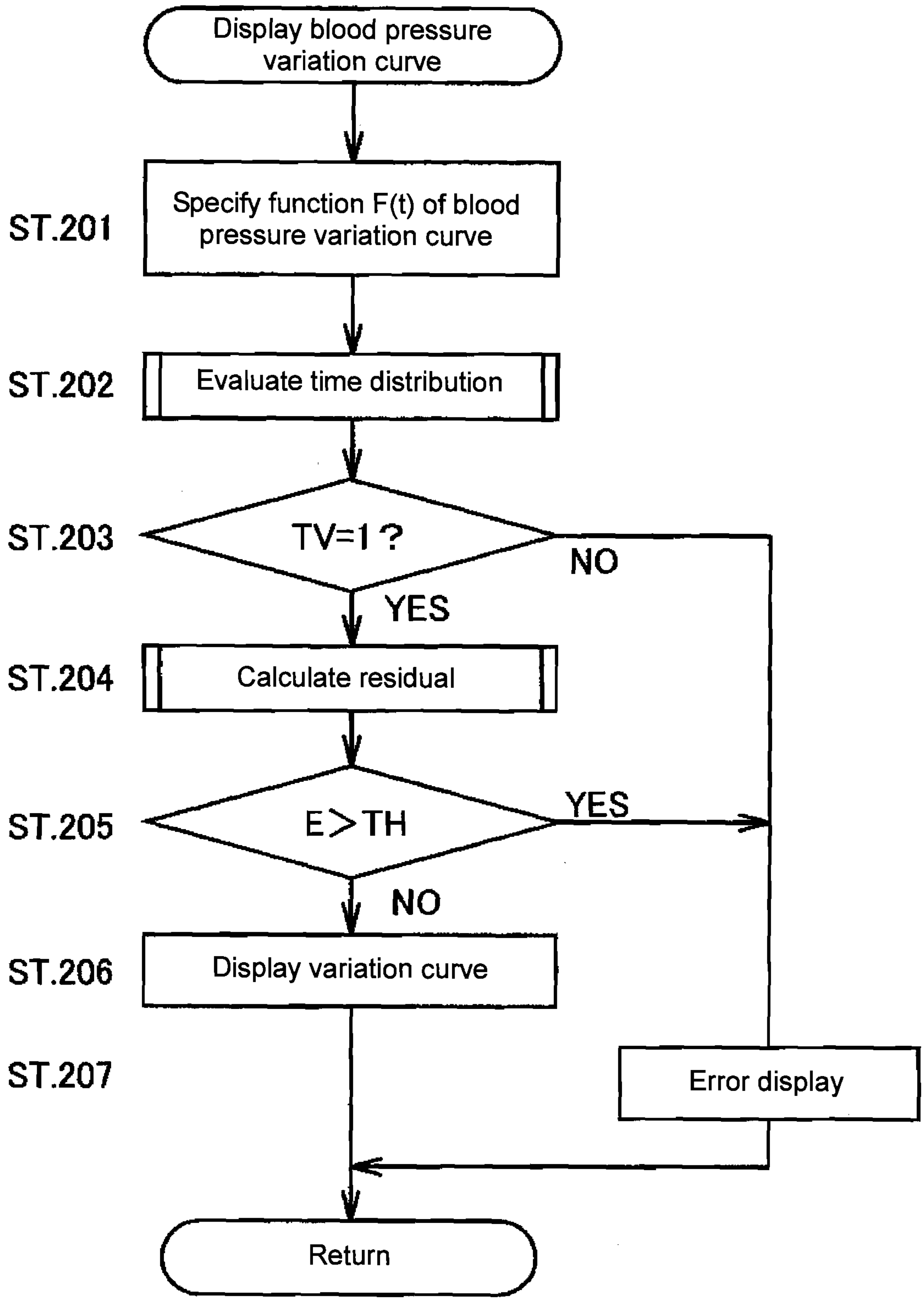
Display blood pressure variation curve
ST.201
Specify function F(t) of blood pressure variation curve
ST.202
Evaluate time distribution
ST.203
TV=1 ?
NO
YES
ST.204
Calculate residual
ST.205
E>TH
YES
NO
ST.206
Display variation curve
ST.207
Error display
Return

Fig. 9

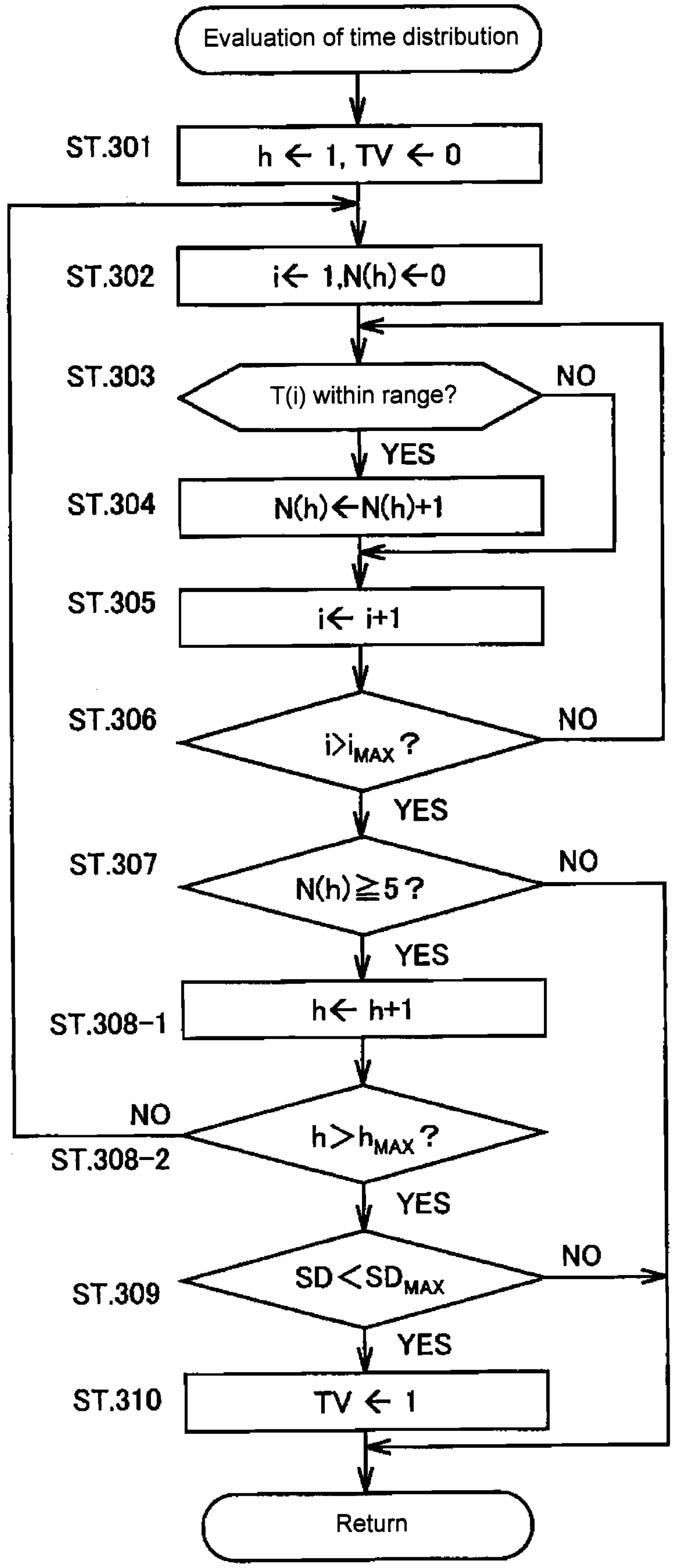
Evaluation of time distribution
ST.301
h ← 1, TV ← 0
ST.302
i← 1,N(h)←0
ST.303
T(i) within range?
NO
YES
ST.304
N(h)←N(h)+1
ST.305
i← i+1
ST.306
i>iMAX ?
NO
YES
ST.307
N(h)≧5 ?
NO
YES
ST.308-1
h← h+1
NO
ST.308-2
h>hMAX ?
YES
ST.309
SD<SDMAX
NO
YES
ST.310
TV ← 1
Return

Fig. 10

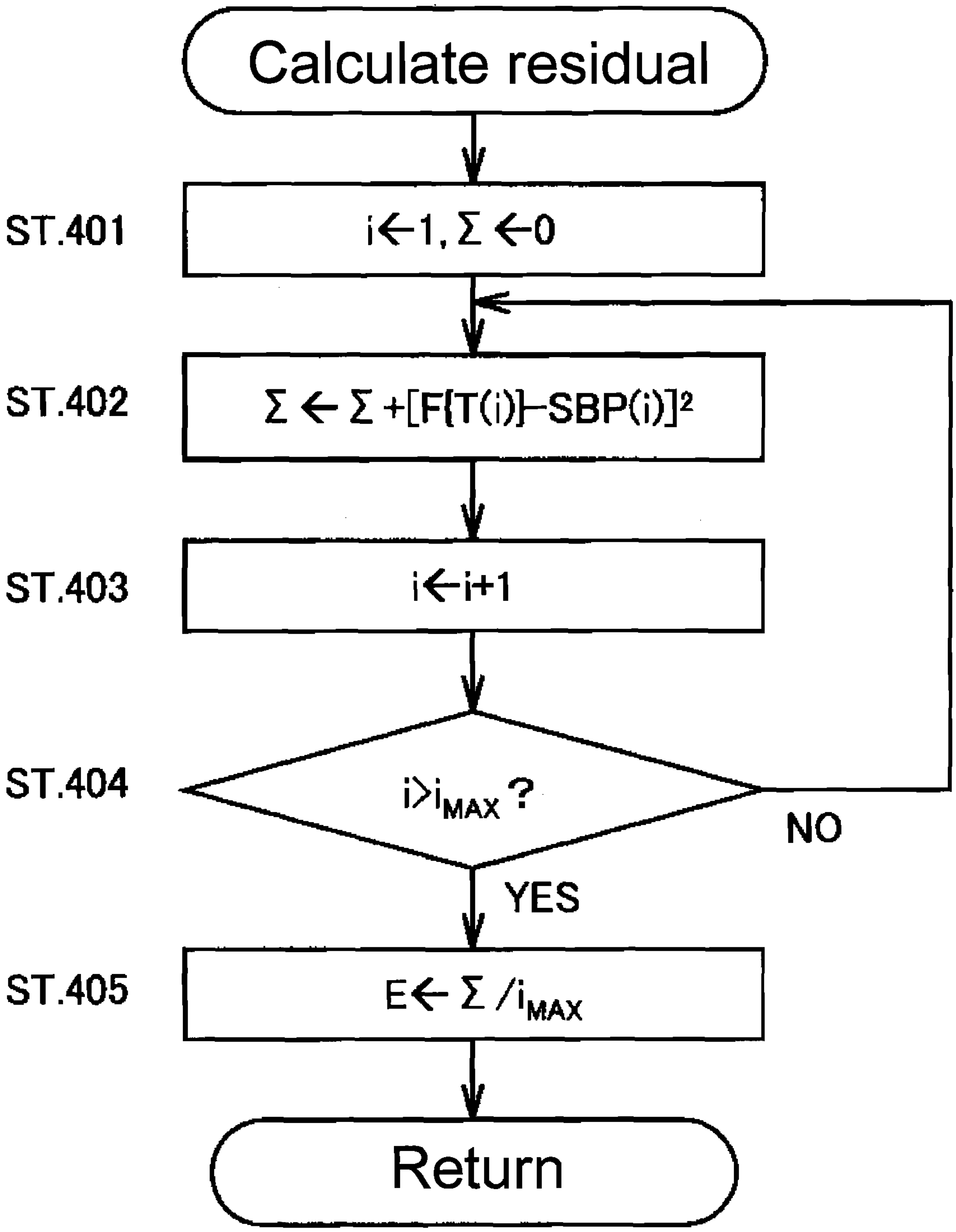
Calculate residual
ST.401
i←1, Σ←0
ST.402
Σ←Σ+[F{T(i)}−SBP(i)]²
ST.403
i←i+1
ST.404
i>i_MAX ?
NO
YES
ST.405
E←Σ/i_MAX
Return

Fig. 11

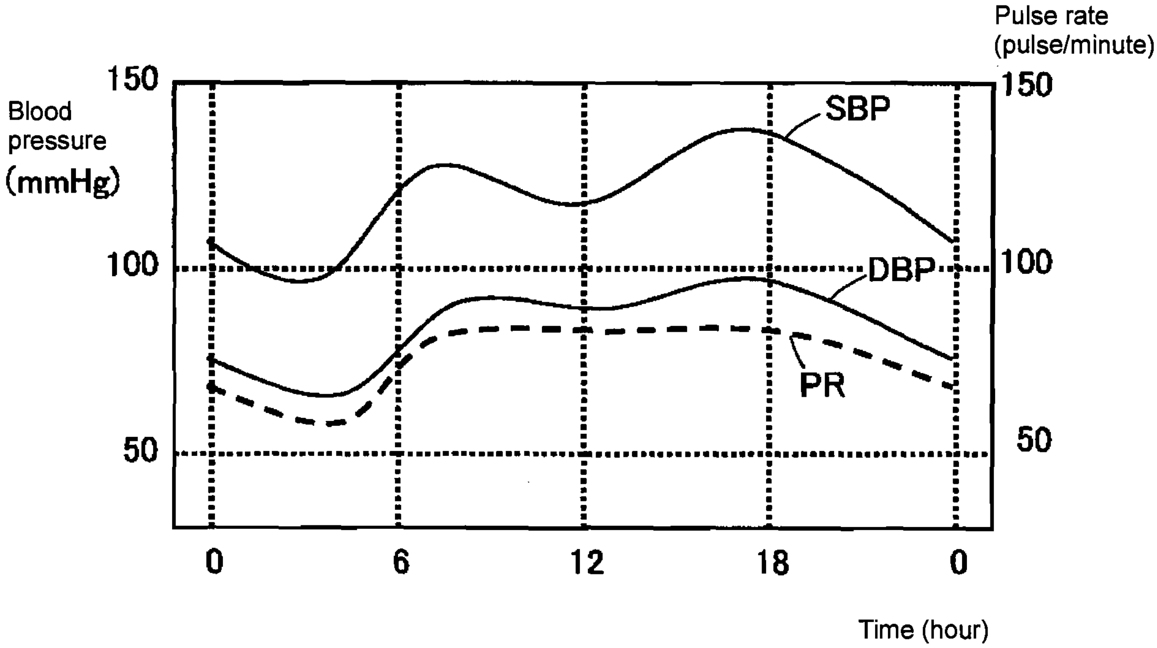
Pulse rate
(pulse/minute)
150
100
50
Blood
pressure
(mmHg)
150
100
50
SBP
DBP
PR
0
6
12
18
0
Time (hour)

Fig. 12A

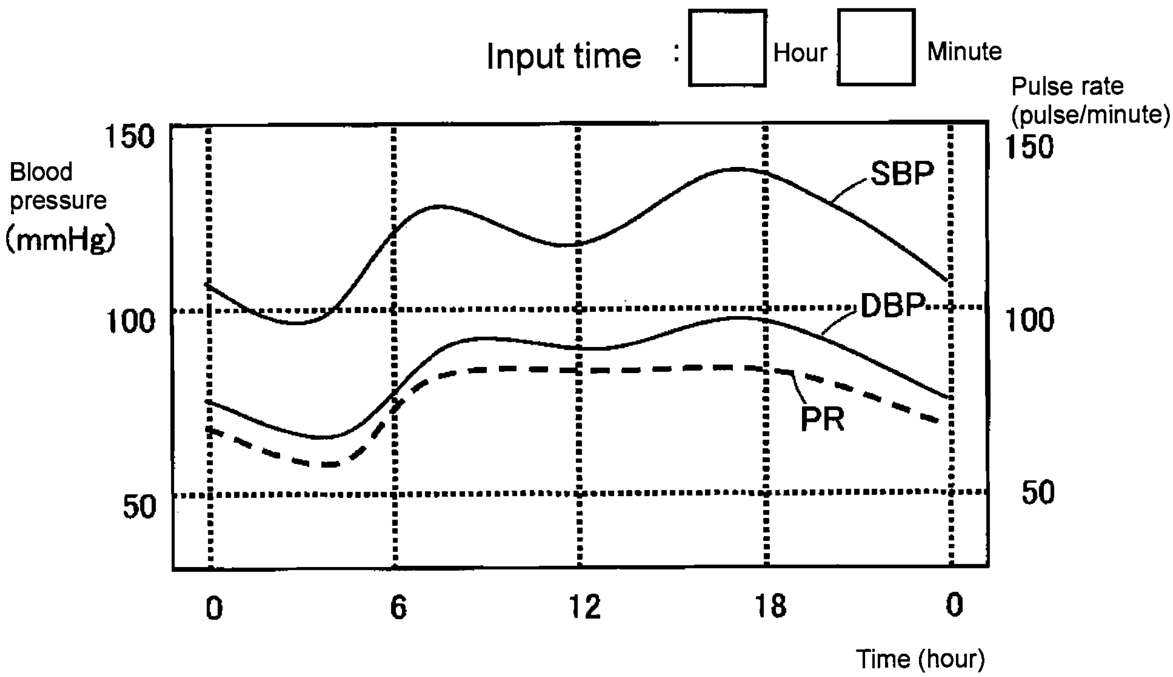
Input time
:
Hour
Minute
Pulse rate (pulse/minute)
Blood pressure (mmHg)
150
100
50
SBP
DBP
PR
0
6
12
18
0
Time (hour)

Fig. 12B

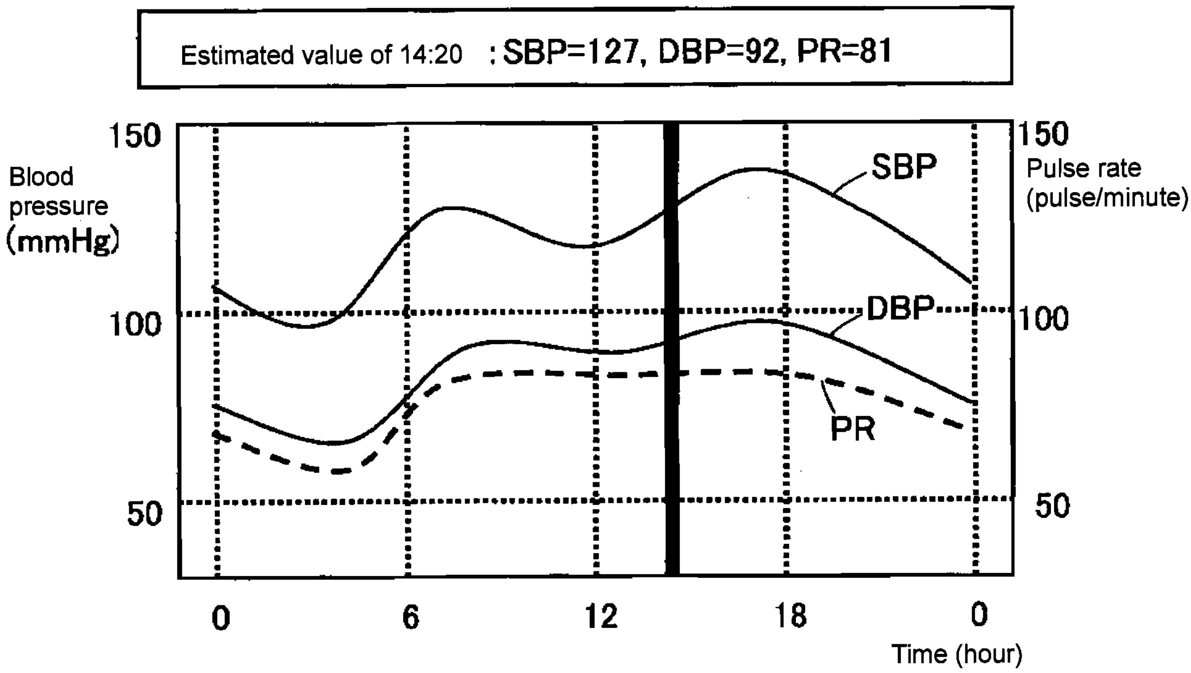
Estimated value of 14:20 : SBP=127, DBP=92, PR=81
150
Blood pressure (mmHg)
100
50
150
Pulse rate (pulse/minute)
100
50
SBP
DBP
PR
0
6
12
18
0
Time (hour)

Fig. 13

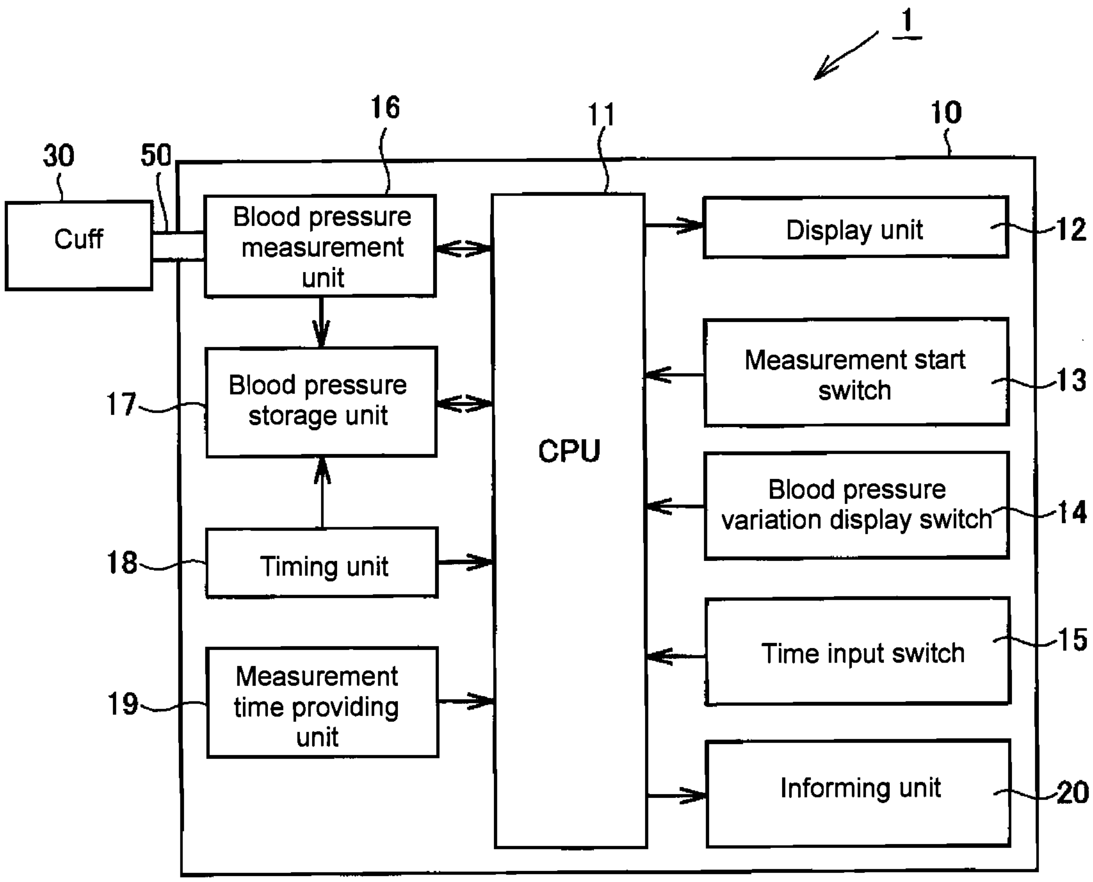
1
16
11
10
30
50
Cuff
Blood pressure measurement unit
Display unit
12
17
Blood pressure storage unit
Measurement start switch
13
CPU
Blood pressure variation display switch
14
18
Timing unit
19
Measurement time providing unit
Time input switch
15
Informing unit
20

Fig. 14

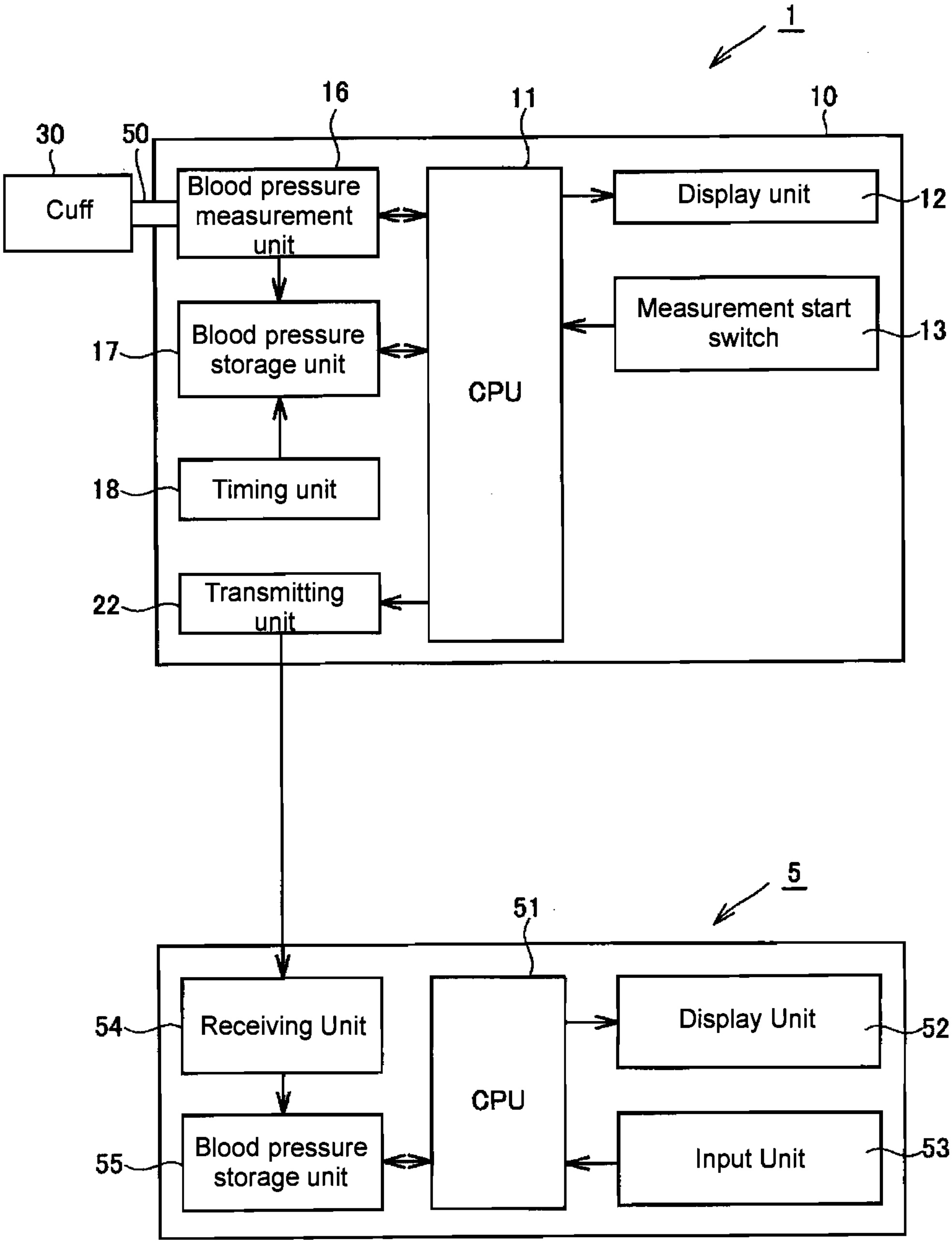
1
30
50
16
11
10
Cuff
Blood pressure measurement unit
Display unit
12
Measurement start switch
13
17
Blood pressure storage unit
CPU
18
Timing unit
22
Transmitting unit
5
51
54
Receiving Unit
Display Unit
52
CPU
55
Blood pressure storage unit
Input Unit
53

**INTERNATIONAL SEARCH REPORT**

International application No.
PCT/JP2007/055872

A. CLASSIFICATION OF SUBJECT MATTER
*A61B5/022*(2006.01)i

According to International Patent Classification (IPC) or to both national classification and IPC

B. FIELDS SEARCHED

Minimum documentation searched (classification system followed by classification symbols)
A61B5/022

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br><br>Y<br><br>A | JP 61-193638 A (Omron Tateisi Electronics Co.), 28 August, 1986 (28.08.86), Full text; all drawings (Family: none) | 1-3,11,15, 18,20<br>4-7,9,12,13, 16,17,19<br>8,10,14 |
| X<br><br>Y<br><br>A | JP 2005-218492 A (Omron Healthcare Co., Ltd., Kazuomi KARIO), 18 August, 2005 (18.08.05), Full text; all drawings & EP 1561419 A2 & US 2005/171442 A1 | 1,2,6,7,9, 11,15,18,20<br>4,5,12,13, 16,17,19<br>8,10,14 |
| Y | JP 6-38936 A (Terumo Corp., Hiroshi HAYASHI), 15 February, 1994 (15.02.94), Full text; all drawings & JP 3231846 B2 | 4,5,12,13,19 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>01 June, 2007 (01.06.07) | Date of mailing of the international search report<br>12 June, 2007 (12.06.07) |
| Name and mailing address of the ISA/<br>Japanese Patent Office<br><br>Facsimile No. | Authorized officer<br><br>Telephone No. |

Form PCT/ISA/210 (second sheet) (April 2005)

**INTERNATIONAL SEARCH REPORT**

International application No.
PCT/JP2007/055872

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2004-261452 A (Omron Healthcare Co., Ltd., Kazuomi KARIO), 24 September, 2004 (24.09.04), Par. Nos. [0121] to [0125]; Fig. 13 & US 2004/176692 A1 | 16 |
| Y | JP 62-66836 A (Sanyo Electric Co., Ltd., Tokyo Sanyo Electric Co., Ltd.), 26 March, 1987 (26.03.87), Page 2, lower left column, line 19 to page 2, lower right column, line 12; Figs. 1, 2 (Family: none) | 17 |

Form PCT/ISA/210 (continuation of second sheet) (April 2005)

**INTERNATIONAL SEARCH REPORT**

International application No.
PCT/JP2007/055872

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The matter common to the inventions of claims 1-20 is equivalent to the matter of claim 1. However, the matter is not novel because the search has revealed that the matter in claims 1-3, 11, 15, 18, and 20 is disclosed in JP 61-193638 A (Omron Tateisi Electronics Co.), 28 August 1986 (28.08.86), full text, all the drawings. (particular reference to page 5, lower right column, line 20 - page 6, lower left column, line 8, and FIG. 4 (10),(11).)

Accordingly, the common matter is not a special technical feature within the meaning of PCT Rule 13.2, second sentence because the matter in (continued to extra sheet)

1. ☒ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (April 2005)

**INTERNATIONAL SEARCH REPORT**

International application No.
PCT/JP2007/055872

Continuation of Box No.III of continuation of first sheet(2)

claims 1-3, 11, 15, 18, and 20 makes no contribution over the prior art.

As a consequence, the inventions of claims 1-20 do not satisfy the requirement of unity of invention.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004261452 A


### Non-patent literature cited in the description


- **MACHIKO TAKE et al.** Reliability of portable blood pressure continuous measurement device ABPM-630 and reproducibility of blood pressure circadian variation. *Journal of Tokyo Women's Medical University,* May 1990, vol. 60 (5), 430-437